# FASCICULE DE BREVET EUROPEEN

(11) **EP 2 243 446 B1**
(45) Date de publication et mention de la délivrance du brevet: **04.07.2012**
(21) Numéro de dépôt: 10159960.3
(22) Date de dépôt: 15.04.2010
(51) Int. Cl.: A61F 5/00, A61F 2/00

(54) **Anneau gastrique nervuré**
Geripptes Magenband
Ribbed gastric ring

(30) Priorité: 21.04.2009 FR 0952604
(43) Date de publication de la demande: 27.10.2010
(73) Titulaire: Compagnie Europeenne d'Etude et de Recherche de Dispositifs pour l'Implantation par Laparoscopie, 38200 Vienne (FR)
(72) Inventeur: Paganon, Pascal, 69360 Serezin du Rhône (FR)
(74) Mandataire: Guérin, Jean-Philippe

(56) Documents cités:
- EP-A- 1 520 564
- WO-A-2004/019671
- FR-A- 2 921 822

## Description

La présente invention se rapporte au domaine général des dispositifs médicaux implantables, et plus particulièrement aux anneaux chirurgicaux, tels que les sphincters ou encore les anneaux gastriques qui sont destinés à être implantés autour de l'estomac ou de l'oesophage dans le cadre d'un traitement de l'obésité.

La présente invention concerne plus particulièrement un anneau chirurgical implantable comportant une bande souple apte à fléchir sur elle même pour former une boucle autour d'un organe biologique constituant une poche ou un conduit afin de modifier la section de passage dudit organe biologique, ladite bande souple comportant une chambre gonflable délimitée par une paroi ventrale destinée à venir au contact de l'organe et une paroi dorsale opposée orientée vers l'extérieur de l'anneau, de telle sorte que ladite chambre se répartit en une portion ventrale et une portion dorsale situées respectivement de part et d'autre d'une surface médiane fictive sensiblement équidistante de la paroi ventrale et de la paroi dorsale.

Il est connu de traiter des patients atteints d'obésité sévère en implantant à ces derniers un anneau gastrique qui vient entourer et serrer l'estomac afin de limiter la prise d'aliments.

De tels anneaux peuvent être posés soit autour de l'estomac intact pour réaliser directement une gastroplastie, soit postérieurement à une opération de « by-pass» au cours de laquelle le chirurgien a préalablement remodelé chirurgicalement l'estomac pour y créer une poche de dimensions réduites.

Dans ce dernier cas, l'anneau a pour objet de s'opposer à la dilatation de la poche gastrique formée chirurgicalement, et prévenir la formation d'une poche plus grande susceptible d'amoindrir l'efficacité thérapeutique de l'intervention.

Bien qu'ils procurent généralement satisfaction, les anneaux de l'art antérieur souffrent parfois de certains inconvénients.

En particulier, la mise en oeuvre de tels anneaux a généralement pour effet de créer, par flexion, un ou plusieurs plis au niveau de la surface ventrale de l'anneau qui vient en contact avec l'organe.

Or l'apparition aléatoire de plis parfois très prononcés peut notamment avoir pour effet de contraindre excessivement la matière constitutive de l'anneau, et par conséquent de faire travailler celui-ci en fatigue au risque de provoquer une diminution de ses propriétés et de ses performances.

Bien entendu, une telle défaillance de l'anneau peut avoir des répercussions importantes sur son effet thérapeutique, en particulier si ledit anneau est pourvu d'une poche gonflable destinée à régler l'effort de serrage que ledit anneau exerce sur l'organe biologique.

De surcroît, la formation de plis peut conduire à une répartition inégale de l'effort de serrage exercé par l'anneau sur l'organe, voire au pincement de la paroi dudit organe, ce qui peut causer une gêne ou une douleur au patient, et même, dans certains cas, provoquer une nécrose des tissus.

Enfin, certains anneaux connus présentent un degré de sophistication important, et notamment des systèmes d'alimentation en fluide de gonflage relativement complexe, ce qui complique leur réalisation, accroît leur coût de fabrication et tend en outre à augmenter leur encombrement global et par conséquent l'inconfort qu'ils génèrent pour le patient.

Le document WO2004/019671 décrit un anneau gastrique selon le préambule de la revendication 1 annexée, notamment muni d'un ballon gonflable présentant plusieurs chambres. Au niveau de sa surface de contact avec l'estomac, le ballon présente plusieurs facettes disposées sensiblement en cercle. Ces facettes sont séparées par des encoches.

Le document EP1520564 décrit un anneau gastrique présentant plusieurs chambres formées dans un ballon. Chaque chambre est alimentée en fluide par l'intermédiaire d'un tube. Le ballon et le tube sont fixés à une ceinture supportant une tension.

Le document FR2921822 décrit un anneau gastrique. Cet anneau comprend des pattes flexibles destinées à éviter son glissement par rapport à l'estomac. L'anneau présente une structure gonflable formant plusieurs chambres munies de facettes de contact avec l'estomac. Les chambres sont séparées par des soufflets.

Les objets assignés à la présente invention visent par conséquent à remédier aux inconvénients énumérés précédemment et à proposer un nouvel anneau chirurgical implantable apte à procurer un serrage efficace, atraumatique et confortable d'un organe biologique, tout en présentant une bonne longévité.

Un autre objet assigné à l'invention vise à proposer un nouvel anneau chirurgical implantable qui présente une structure particulièrement simple, compacte, légère, et qui soit peu onéreux à fabriquer.

Un autre objet assigné à l'invention vise à proposer un nouvel anneau chirurgical implantable ajustable et polyvalent.

Un autre objet assigné à l'invention vise à proposer un nouvel anneau chirurgical implantable qui présente un comportement prévisible, contrôlable, et reproductible, ainsi qu'une bonne réponse dynamique.

Un autre objet assigné à l'invention vise à proposer un anneau chirurgical implantable dont la pose soit facilitée.

Enfin, un autre objet assigné à l'invention vise à proposer un nouvel anneau chirurgical implantable qui procure un serrage homogène de l'organe biologique.

Les objets assignés à l'invention sont atteints à l'aide d'un anneau chirurgical implantable tel que défini dans la revendication 1 ou l'une de ses revendications dépendantes

D'autres objets caractéristiques et avantages de la présente invention apparaîtront plus en détails à la lecture de la description qui suit, ainsi qu'à l'aide des dessins annexés, fournis à titre purement illustratif et non limitatif, parmi lesquels :
- La figure 1 illustre, selon une vue en perspective, un anneau chirurgical implantable en configuration ouverte.
- La figure 2 illustre, selon une vue latérale, l'anneau chirurgical de la figure 1.
- La figure 3 illustre, selon une vue en perspective avec arrachement de matière au niveau du plan sagittal, une variante de réalisation d'un anneau tel que celui représenté sur les figures 1 et 2.
- La figure 4 illustre une section transverse de l'anneau chirurgical représenté sur la figure 3.
- La figure 5 illustre, selon une vue analogue à celle de la figure 3, une autre variante de réalisation d'un anneau chirurgical.
- La figure 6 illustre une section transverse de l'anneau chirurgical représenté sur la figure 5.
- Les figures 7A, 7B et 7C illustrent, selon des vues en section, plusieurs variantes de réalisation de moyens de renfort.
- La figure 8 illustre, selon une vue en perspective, un anneau chirurgical conforme à l'invention, en configuration fermée.
- La figure 9 illustre une section transverse de l'anneau chirurgical représenté sur la figure 8.

La présente invention concerne de manière générale un anneau chirurgical implantable 1 destiné à être placé autour d'un organe biologique (non représenté) constituant une poche ou un conduit, en vue de modifier la section de passage dudit organe biologique.

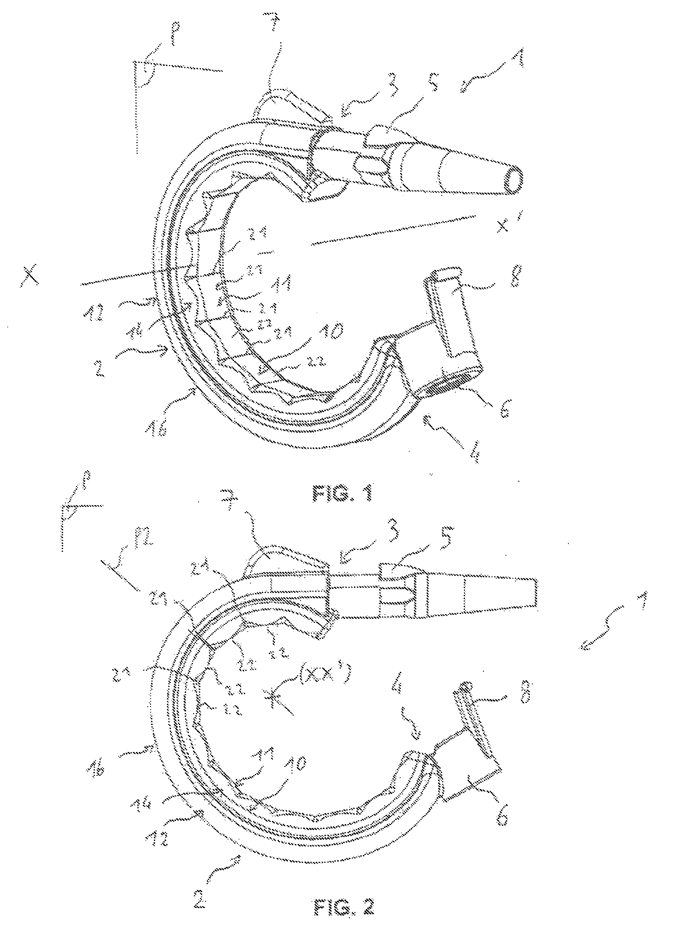

Les variantes illustrées aux figures 1 à 7, bien que non couvertes par les revendications, sont fournies pour mieux illustrer l'invention .

Selon une variante de réalisation, ledit anneau 1 peut être agencé pour former un sphincter destiné à la régulation du flux sanguin ou au traitement de l'incontinence urinaire ou fécale.

Toutefois, de façon préférentielle, l'anneau 1 constitue un anneau gastrique destiné au traitement de l'obésité, qui est conçu pour être positionné sur l'oesophage ou sur l'estomac.

De façon particulièrement préférentielle, l'anneau 1 est spécifiquement adapté pour être placé sur la poche stomacale qui résulte d'une opération chirurgicale de by-pass, afin de s'opposer à la dilation post-opératoire de ladite poche stomacale, et constitue ainsi un anneau de by-pass gastrique.

Selon l'invention, l'anneau 1 comporte une bande souple 2 qui est apte à fléchir sur elle-même pour former une boucle autour de l'organe, et plus particulièrement à s'enrouler autour d'un axe (X-X').

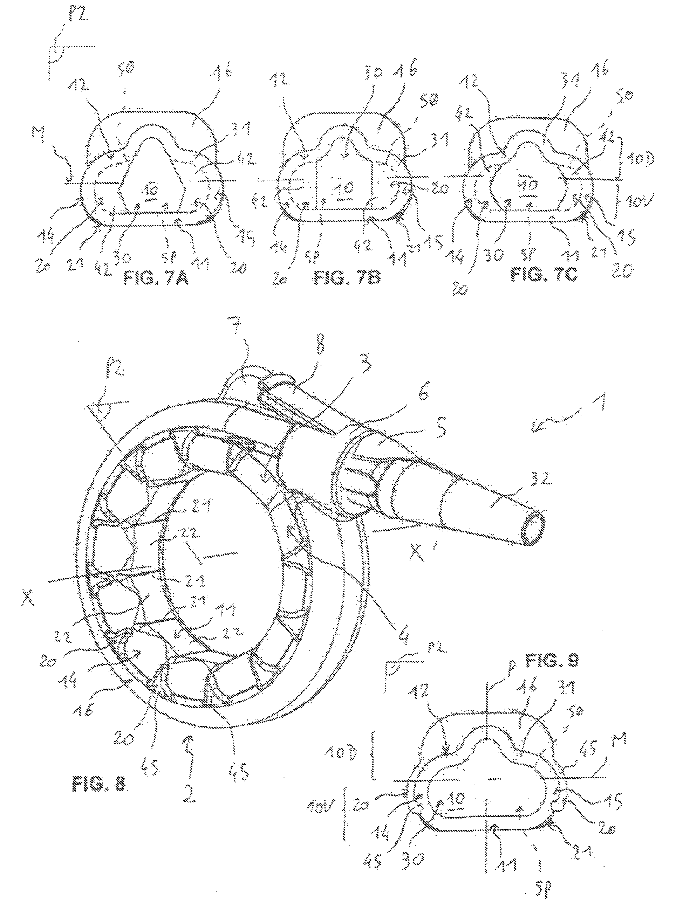

Bien qu'il soit envisageable, sans sortir du cadre de l'invention, que la bande souple 2 forme une sorte de selle ouverte destinée à entourer partiellement l'organe concerné, par exemple en forme de «U», ladite bande souple 2 sera de préférence suffisamment longue pour contenir sensiblement tout le périmètre de l'organe biologique, et notamment pour être fermée sur elle-même, de préférence sensiblement au niveau de ses extrémités 3, 4, de sorte à pouvoir former une boucle close entourant l'organe, tel que cela est illustré sur la figure 8.

A cet effet, la bande souple est préférentiellement pourvue de moyens de verrouillage 5, 6 conçus pour la maintenir en configuration fermée, lesdits moyens de verrouillage comprenant par exemple un ou plusieurs éléments mâles 5, tels qu'un ergot, destinés à coopérer, par exemple par encliquetage, avec un élément femelle 6 conjugué, tel qu'un manchon.

Avantageusement, les moyens de verrouillage 5, 6 sont réversibles, de telle sorte que l'anneau 1 puisse passer alternativement, sous la commande du praticien d'une configuration ouverte dans laquelle ses extrémités 3, 4 sont séparées et distantes, tel que cela est illustré par exemple sur les figures 1 et 2, à une configuration fermée dans laquelle ses extrémités 3, 4 sont réunies et maintenues au contact l'une de l'autre, tel que cela est illustré sur la figure 8.

Bien entendu, le contour délimité par l'anneau 1 conforme à l'invention autour de l'organe biologique n'est nullement limité à une forme particulière.

Toutefois, l'anneau 1 sera de préférence conçu pour adopter, lorsqu'il se trouve en configuration armée, la forme d'une boucle sensiblement circulaire d'axe (X-X').

Par ailleurs, l'anneau 1 peut comporter une ou plusieurs languettes de préhension 7, 8, destinées à faciliter la manipulation dudit anneau 1, notamment lors de son implantation, et/ou à permettre l'actionnement des moyens de verrouillage 5, 6.

Tel que cela est illustré sur les figures, la bande souple 2 comporte une chambre gonflable 10 qui est délimitée, au plus près de l'axe (X-X'), par une paroi ventrale 11 destinée à venir au contact de l'organe biologique, une paroi dorsale 12 opposée orientée vers l'extérieur de l'anneau, dans la zone la plus éloignée de l'axe (X-X'), ainsi que par une première paroi latérale 14 et une seconde paroi latérale 15 qui joignent la paroi ventrale 11 à la paroi dorsale 12 de part et d'autre de la chambre selon l'axe (XX'), de préférence selon des directions sensiblement radiales.

Ainsi, ladite chambre 10 se répartit en une portion ventrale 10V et une portion dorsale 10D situées respectivement de part et d'autre d'une surface médiane M fictive sensiblement équidistante de la paroi ventrale 11 et de la paroi dorsale 12.

De préférence, le rebord marquant la transition entre la face ventrale 10 et les faces latérales 14, 15 présente un contour arrondi et atraumatique.

Avantageusement, la paroi ventrale 11 est conçue pour venir au contact dudit organe de manière à enserrer ce dernier, selon un diamètre de constriction qui peut être ajusté en fonction de la quantité de fluide de remplissage, tel que de l'air ou du sérum physiologique, contenue dans la chambre 10.

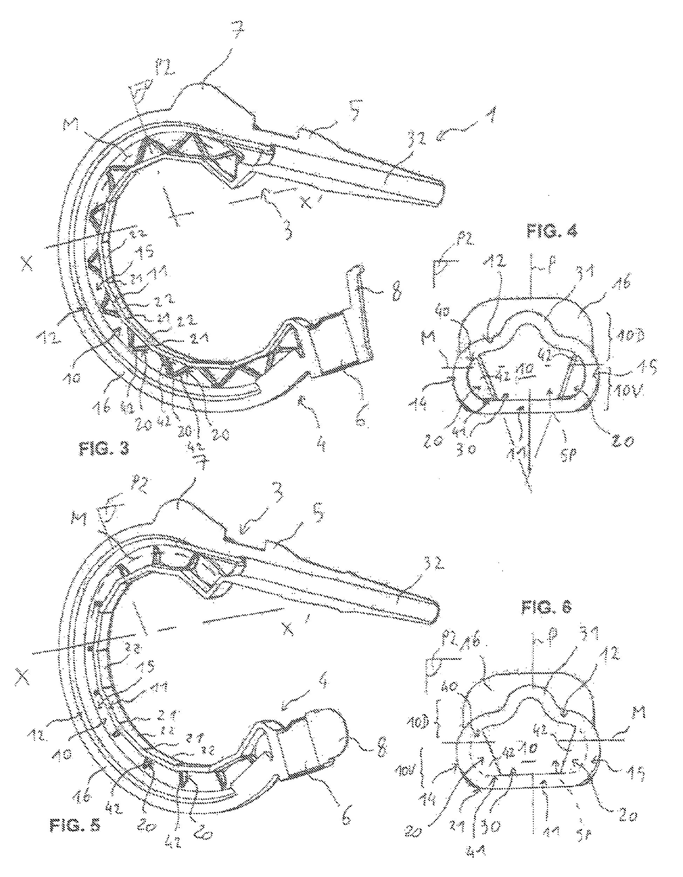

De préférence, tel que cela est illustré notamment sur la figure 5, la paroi dorsale 12 est accolée à, voire formée par une ceinture périphérique 16 qui est de préférence sensiblement inextensible de sorte à former un organe de support, dont le périmètre est sensiblement invariant une fois l'anneau fermé.

En outre, la chambre 10 s'étend de préférence en longueur sensiblement sur l'ensemble du périmètre défini par la ceinture dorsale 16, de la première extrémité 3 à la seconde extrémité 4 de la bande souple 2.

Selon une caractéristique préférentielle de l'invention, la bande souple 2 est préformée de telle sorte que la face ventrale 10 est incurvée autour de l'axe (X-X').

Avantageusement, en conférant à la bande souple 2 une forme au repos qui présente une certaine courbure qui « amorce» la flexion de ladite bande souple sur elle-même autour de l'axe (X-X'), on facilite la mise en place de l'anneau 1 autour dudit organe, et notamment sa fermeture par rapprochement de ses extrémités 3, 4.

Bien entendu, la courbure initiale au repos de ladite bande souple 2 pourra être librement choisie par l'homme du métier lors de la fabrication, et plus ou moins prononcée.

Selon une caractéristique importante de l'invention, l'anneau comporte une pluralité de moyens de renfort 20 reliant chacun la portion ventrale 10V de la chambre à la portion dorsale 10D de celle-ci afin de limiter le débattement de la paroi ventrale 11 par rapport à la paroi dorsale 12 lors du gonflage de la chambre 10, et ainsi créer au niveau de la paroi ventrale des zones de pliage 21 prédéfinies qui subdivisent ladite chambre en une pluralité de tronçons 22 contigus. Cette disposition permet d'obtenir une forme géométrique de serrage qui est circulaire et homogène sur la majorité voire la totalité de la plage d'ajustement. On évite ainsi l'apparition de zones de pincement et/ou de points de pression sources de traumatisme pour les tissus de l'estomac ou des organes biologiques.

Avantageusement, l'agencement conforme à l'invention permet de discrétiser le contour interne de l'anneau 1, au niveau de la paroi ventrale 10, en une chaîne longitudinale de tronçons 22 successifs, de préférence située sensiblement à la même abscisse par rapport à l'axe (X-X'), qui s'articulent les uns par rapport aux autres de manière reproductible et prévisible, au niveau de zones de pliage 21 préférentielles pré-formées par construction.

Un tel agencement permet avantageusement de contrôler l'emplacement et l'amplitude de formation des plis lorsque l'anneau 1 est mis en place autour de l'estomac, et ainsi de limiter la fatigue subie par le matériau constitutif de la bande souple 2, et plus particulièrement de la paroi ventrale 11.

En outre, les zones de pliage 21 sont de préférence particulièrement étroites, de telle sorte que leur couverture angulaire, c'est-à-dire l'angle correspondant à l'arc formé par une zone de pliage 21 dans un plan E normal à l'axe (X-X'), et dont le sommet se situe sur ledit axe (X-X'), tel que cela est illustré sur la figure 2, est faible, et de préférence inférieure ou égale à 5°, voire inférieure ou égale à 3°, voire inférieure ou égale à 1°.

Par commodité de description, on considérera que le plan P est formé par le plan sagittal de l'anneau 1, perpendiculaire à l'axe (XX') et coupant l'anneau en son milieu, sensiblement à égale distance de chacune des parois latérales 14, 15.

Selon une variante de réalisation préférentielle, les tronçons 22 forment, au niveau de la paroi ventrale 11, des facettes sécantes, et les zones de pliage 21 sont formées par des arêtes qui correspondent à l'intersection des facettes contiguës, tel que cela est illustré sur les figures 1, 2, 3, 5 et 8.

Il est par ailleurs remarquable que la combinaison préférentielle d'une pré-forme courbe et d'une division en facettes tel que revendiqué permet d'optimiser le contrôle des zones de pliage, et de limiter la fatigue subie par le matériau constitutif de la bande souple 2, et plus particulièrement de la paroi ventrale 11.

Par commodité de description, on fera référence aux tronçons 22 dans leur ensemble ainsi qu'aux zones de pliage 21 dans leur ensemble. Toutefois, il est parfaitement envisageable, sans sortir du cadre de l'invention, que l'une ou l'autre des caractéristiques décrites puissent s'appliquer à un tronçon ou une zone de pliage en particulier, ou encore à la majorité ou à la totalité des tronçons et/ou des zones de pliage. Bien entendu, certains tronçons et zones de pliage peuvent présenter des caractéristiques propres qui les distinguent des autres tronçons ou des autres zones de pliage.

De préférence, les zones de pliage 21 sont particulièrement étroites et peu profondes. De préférence, elles seront linéaires et sensiblement parallèles à l'axe (X-X'), résultant directement de l'accolement bord à bord des deux tronçons 22 qui leur sont adjacents et qui forment de préférence un angle vif.

En d'autres termes, les zones de pliage se résument de préférence, aux congés de raccordement près, à des traits rectilignes joignant la première face latérale 14 à la seconde face latérale 15 et marquant la limite de séparation entre les tronçons 22 successifs.

Bien entendu, le nombre, la forme et les dimensions des tronçons 22 ainsi que des zones de pliage qui sont interposées entre ces derniers peuvent faire l'objet de variations sans sortir du cadre de l'invention.

Toutefois, l'anneau 1 conforme à l'invention comportera de préférence au moins 4 tronçons, au moins 6 tronçons, voire 12 tronçons, tel que cela est illustré sur les figures 1, 2, 3, 5 et 8.

Chaque tronçon 22 pourra présenter une étendue relativement importante, de sorte à fournir une surface d'appui sensiblement atraumatique et à limiter les risques de pincement de la paroi de l'organe biologique.

Plus particulièrement, chaque tronçon couvrira de préférence un secteur angulaire, mesuré autour de l'axe (X-X') et dans le plan P, sensiblement compris entre 10° et 90°, de préférence sensiblement compris entre 20° et 60° et de façon particulièrement préférentiel sensiblement compris entre 15° et 35°.

En outre, la paroi ventrale 10 présentera de préférence sur toute sa longueur, c'est-à-dire entre la première extrémité 3 de la bande souple 2 et la seconde extrémité 4 de cette dernière, une succession ininterrompue de tronçons et de zones de pliage alternées.

De préférence, la répartition des tronçons sera régulière le long de la face ventrale 10. Plus particulièrement, lesdits tronçons pourront être équirépartis et orientés de telle sorte que leurs médiatrices respectives convergent vers l'axe (X-X') et se coupent sensiblement au niveau dudit axe.

Selon une variante de réalisation préférentielle, les tronçons 22 sont sensiblement identiques les uns aux autres, et notamment peuvent présenter sensiblement la même couverture angulaire, les mêmes dimensions longitudinale et transverse.

Ainsi, la face ventrale 10 peut avantageusement former, lorsque l'anneau se trouve en configuration fermée, un polyèdre régulier dont les faces sont de préférence sensiblement parallèles à l'axe (X-X').

Un tel agencement permet avantageusement d'obtenir un serrage homogène de l'organe biologique.

Par ailleurs, la paroi ventrale forme de préférence au niveau de chaque tronçon 22 une facette sensiblement plane, lorsque l'anneau chirurgical se trouve au repos, c'est-à-dire lorsque la chambre 10 n'est pas déformée sous la contrainte d'un fluide sous pression.

Ainsi, le contour au repos de la paroi ventrale 11, considéré dans le plan P se présentera de préférence sensiblement sous la forme d'une ligne polygonale brisée, et plus particulièrement d'un polygone régulier lorsque l'anneau est en configuration fermée. En d'autres thermes la paroi ventrale 11 dessine de préférence, en projection dans le plan P, un polygone régulier inscriptible dans un cercle centré sur l'axe (X-X'), dont les sommets sont formés par les projections des arêtes et dont les côtés, correspondant aux cordes qui relient entre eux les sommets situés sur ledit cercle, sont formés par les projections des facettes.

Avantageusement, cet agencement particulièrement compact, dans lequel chaque facette se trouve sensiblement dans le prolongement de la précédente, facilite l'introduction de l'anneau autour de l'organe, en particulier lorsqu'il est nécessaire de l'engager dans l'espace rétro-gastrique.

En outre, une telle géométrie externe, particulièrement simple, est favorable à la réalisation de l'anneau 1 par moulage, sur un noyau polyédrique.

Selon une autre caractéristique importante de l'invention, les moyens de renfort 20 sont agencés pour conserver entre les tronçons 22 contigus des orifices de passage 30 permettant la libre circulation de fluide de gonflage et dont la section SP est supérieure ou égale à 10 %, de préférence supérieure ou égale à 30%, et de façon particulièrement préférentielle supérieure ou égale à 50% voire supérieure ou égale à 75 % de la section SO desdits tronçons 22.

Ainsi, avantageusement, les moyens de renfort 20 sont aptes à renforcer et marquer les plis sans faire obstacle au libre remplissage rapide et homogène de l'ensemble de la chambre 10.

En effet, les inventeurs ont constaté qu'il était possible d'opter pour des moyens de renfort de dimensions relativement modestes et d'obtenir malgré tout un marquage efficace des zones de pliages, par une retenue active de la paroi ventrale 11 par rapport aux portions libres des tronçons 22 qui sont agencées pour se déplacer, de préférence par déformation élastique, selon une direction radiale centripète, et ce sans sacrifier la libre circulation de fluide au sein de la chambre 10.

En outre, la mise en oeuvre de moyens de renfort 20 qui préservent un passage 30 de section SP, et notamment de largeur, significative, voire correspondant à plus de la moitié de la largeur maximale de la chambre 10, permet de conférer à l'anneau 1 conforme à l'invention une structure particulièrement légère, souple et dont la fabrication par moulage est grandement simplifiée.

A ce titre, il est remarquable que la chambre 10 peut avantageusement être moulée d'un seul tenant avec des moyens de renfort 20 incorporés, en mettant en oeuvre un noyau monobloc et en jouant simplement sur l'élasticité intrinsèque du matériau constitutif des parois de ladite chambre 10.

Bien entendu, les proportions susmentionnées, et plus globalement les propriétés géométriques et dimensionnelles des constituants de l'anneau, sont de préférence mesurées au repos, mais évoluent de préférence sensiblement dans les plages de valeurs indiquées lors des modifications de configuration provoquées par le gonflage ou le dégonflage de l'anneau, et notamment lorsque la chambre est déformée par gonflage.

De préférence, la chambre gonflable 10 peut être délimitée par une membrane 31 sensiblement tubulaire et réalisée dans un matériau élastomère biocompatible du genre silicone.

De préférence, ladite membrane 31 forme d'un seul tenant la paroi ventrale 11, la paroi dorsale 12, ainsi que la première et la seconde parois latérales 14, 15, tel que cela est illustré sur les figures 4, 6, 7A à 7C et 9, ce qui contribue à améliorer sa tenue mécanique et son étanchéité.

La ceinture dorsale 16 peut avantageusement être accolée le long de la paroi dorsale 12, de préférence à l'extérieur de la chambre 10, par exemple par collage ou par tout autre moyen d'assemblage équivalent.

Avantageusement, la chambre gonflable 10 communique avec un embout 32 permettant l'apport ou le retrait de fluide dans ladite chambre, par exemple au moyen d'un cathéter relié à un réservoir de fluide déporté (non représentés).

Bien entendu, il serait toutefois envisageable de mettre en oeuvre l'agencement propre à l'invention au sein d'un anneau dont la chambre serait scellée et par exemple remplie d'un volume prédéterminé d'un gaz compressible.

De préférence, la paroi ventrale 10 est distincte de la paroi dorsale et est détachée de cette dernière de sorte à se trouver à distance de celle-ci en permanence, sensiblement sur l'ensemble du périmètre de la bande souple 2, y compris lorsque l'anneau se trouve au repos, afin d'assurer une communication continue entre l'ensemble des tronçons 22.

Selon une variante de réalisation préférentielle, tel que cela est illustré sur les figures 4, 6, 7A à 7C et 9, la section des tronçons 22, et plus particulièrement la section transverse de la chambre 10 considérée dans un plan de coupe P2 contenant l'axe (XX'), peut présenter plusieurs lobes, et par exemple trois lobes formant sensiblement une feuille de trèfle.

De préférence, la chambre 10 comporte en outre une tranchée 33 qui forme un renfoncement de la paroi dorsale 12 creusé dans la ceinture dorsale 16. Cette tranchée, de préférence annulaire et orientée sensiblement normalement à l'axe (XX'), forme une sorte de charnière qui améliore le déploiement de la paroi ventrale 11, en tendant à provoquer un certain écartement antagoniste, un peu à la manière d'une corolle, de la première et de la seconde paroi latérale 14, 15. Un tel comportement limite avantageusement la formation de plis à la surface de la membrane 31, en particulier au niveau de la paroi ventrale 11.

Avantageusement, les moyens de renfort 20 sont conçus pour agir sur les zones de pliage 21 afin de s'opposer à leur déplacement radial, ici centripète, lorsque les tronçons 22 formant les portions libres de la paroi ventrale 11 qui leur sont voisines sont entraînées en déplacement radial, à l'occasion du gonflage de la chambre 10.

Bien que cet effet de retenue puisse être obtenu par une répartition suffisante, c'est à dire une portée suffisamment étendue, de chaque moyen de renfort 20 de part et d'autre de la surface médiane M, par exemple par un renfort situé dans la zone intermédiaire des parois latérales 14, 15, les moyens de renfort 20 formeront de préférence des tirants qui joignent la paroi ventrale 11 à la paroi dorsale 12.

Ainsi, tel que cela est illustré sur les figures 3 à 7C, la paroi ventrale sera de préférence directement arrimée, par des éléments de jonction mécanique, à la paroi dorsale, elle- même retenue par la ceinture dorsale 16.

En conférant à chaque moyen de renfort 20 un pied 40 qui prend racine au niveau de la paroi dorsale et un chapiteau 41 qui le relie à la paroi ventrale, on améliore sensiblement l'assise et la résistance à la traction, ainsi qu'à la rupture, des tirants. Ceux-ci présenteront ainsi une excellente robustesse lorsqu'ils s'opposeront directement à la sollicitation en traction radiale lors du gonflage.

Bien entendu, les moyens de renfort 20 conformes à l'invention ne sont nullement limités dans leur nature ou leur géométrie et pourront faire l'objet de variations sans sortir du cadre de l'invention tel que défini par les revendications, dès lors qu'ils constitueront des structures créant localement au niveau de la chambre, et plus particulièrement au niveau des parois latérales, des zones d'hétérogénéité moins sujettes à la déformation radiale que les autres zones de la chambre.

Ainsi, la rigidité des moyens de renfort 20 pourra être obtenue par la sélection de leur matériau constitutif, qui sera plus rigide que celui de la membrane 31, ou encore par l'emploi d'inserts ou la mise en oeuvre de surépaisseurs de matière dans les zones concernées.

Selon une variante de réalisation non représentée, les moyens de renfort 20 pourront être formés par des piliers qui joignent directement la paroi ventrale 11 à la paroi dorsale 12 sans toucher les parois latérales 14, 15, en ménageant ainsi, entre eux et lesdites parois latérales 14, 15 un passage 30 prenant la forme de deux ouïes.

De préférence de tels piliers pourront être centrés dans la chambre 10, en chevauchant le plan sagittal P. La paroi ventrale pourra alors extérieurement adopter un aspect capitonné, creusé sur son pourtour central d'une pluralité de dépressions correspondant aux chapiteaux 41 desdits piliers.

Toutefois, les moyens de renfort 20 comportent, voire sont exclusivement formés, par des nervures 42 formant des surépaisseurs venues de matière avec les parois latérales 14, 15 de la chambre 10, tel que cela est illustré sur les figures 3 à 7C.

En outre, selon une variante de réalisation, les moyens de renfort 20 s'étendent de préférence en saillie à l'intérieur de la chambre 10, en empiétant sur la section SO de ladite chambre mais sans toutefois obstruer celle-ci.

Lesdites nervures formeront avantageusement des renflements à la surface interne de la membrane 31, cette dernière étant de préférence d'épaisseur sensiblement constante par ailleurs.

Ainsi, selon une variante de réalisation correspondant aux figures 3 à 7C, lesdites nervures 42 sont formée par des plaques, éventuellement voilées au repos, qui sont fixées à la fois à la paroi ventrale 10 au niveau de leur chapiteau 41, à la paroi dorsale, au niveau de leur pied 40, et à une paroi latérale 14, 15 au niveau de leur chant, et cloisonnent ainsi partiellement la section de la chambre 10.

De préférence, lesdites plaques sont aboutées à la paroi latérale, et de préférence venues de matière avec cette dernière, par leur bord de plus faible épaisseur, et s'étendent à la manière d'ailettes à travers la chambre selon leurs deux dimensions principales. De préférence, lesdites plaques s'étendent en longueur sur la totalité de la hauteur de la chambre 10, selon une direction radiale par rapport à l'axe (XX'), et en largeur sur une partie de la largeur des parois ventrale 11 et dorsale 12, selon une direction sensiblement tangente au périmètre de la bande souple 2.

De préférence, les nervures 42, et plus particulièrement les plaques, s'étendent sensiblement perpendiculairement à la paroi latérale 14, 15 à laquelle elles sont rattachées.

Bien entendu, les plaques peuvent varier dans leurs formes et leurs dimensions, dès lors qu'elles contribuent à la formation des plis sans entraver la circulation de fluide au sein de la chambre 10. Ainsi, elles pourront présenter, au niveau de leur bord libre, des contours rectilignes (figures 4, 6 et 7B), polygonaux (figure 7A), bombés, de préférences concaves, et notamment arrondis en croissant (figure 7C).

Par ailleurs selon une caractéristique commune aux variantes de réalisation des figures 3 à 9, seule ou en combinaison avec l'une ou l'autre des caractéristiques présentement décrites, les moyens de renfort 20 sont de préférence disposés par paire, de part et d'autre de la chambre 10.

Plus particulièrement, les moyens de renfort 20 sont de préférence appariés de telle sorte que chacun des organes d'une même paire agisse séparément à l'une des deux extrémités latérales d'une même zone de pliage 21, de sorte à équilibrer la formation du pli selon le travers de la paroi ventrale 11 selon une direction de préférence sensiblement parallèle à l'axe (XX').

Avantageusement, chaque arête sera ainsi pilotée par au moins deux tirants appariés qui la retiennent l'un à gauche, l'autre à droite du plan sagittal P.

De façon particulièrement préférentielle, les moyens de renfort 20 sont disposés de manière sensiblement symétrique par rapport au plan sagittal P de la chambre 10, la chambre 10 et l'anneau 1 dans son ensemble présentant de préférence une symétrie par rapport audit plan P.

Avantageusement, les nervures 42 occulteront sensiblement les lobes latéraux de la chambre 10, tout en dégageant au milieu de ladite chambre un passage 30 central, de préférence formé par une bande de section rectangulaire (figure 7B) polygonale (figure 7A), circulaire ou elliptique (figure 7C) ou trapézoïdale (figure 6).

Selon une variante de réalisation illustrée sur la figure 4, les bords libres des nervures 42 qui se font face au niveau d'une même paire sont inclinés et convergent l'un par rapport à l'autre de sorte à former un angle, de préférence sensiblement compris entre 30° et 60°, d e préférence voisin de 45° et dont le sommet, de préférence fictif, et situé à l'intérieur de la boucle, dans le plan P. Ainsi, le chapiteau 41 desdites nervures 42 sera de préférence plus large que leur pied 40.

Une telle disposition permet notamment d'élargir le passage 30 à sa base afin de faciliter la circulation du fluide, mais également de simplifier la réalisation du noyau de moulage.

Par ailleurs, selon une variante de réalisation correspondant aux figures 5 et 6, les moyens de renfort 20 s'étendent sensiblement perpendiculairement à la face ventrale 11 et à la face dorsale 12, c'est à dire sont sensiblement portés par des plans radiaux, répartis en étoile autour de l'axe (XX').

Toutefois, selon une autre variante de réalisation correspondant aux figures 3 et 4, les moyens de renfort 20 sont disposés en oblique de sorte à former des structures triangulaires dont les sommets coïncident avec les zones de pliage 21 .

Ainsi, la chambre 10 peut avantageusement être renforcée par une structure de poutres montées en opposition par leurs sommets, de type «cantilever» qui zigzague alternativement de la paroi ventrale 11 à la paroi dorsale 12 et vient «haubaner» chaque arête au moyen de deux paires de tirants formant un triangle dont la base est fixée à la paroi dorsale 12 et dont le sommet coïncide avec l'arête.

Une telle structure confère avantageusement une excellente répartition des contraintes de flexion tout au long de la chambre 10, et permet d'obtenir un pliage précis et homogène, dans les zones de pliages 21 où se rejoignent deux à deux les tirants, sans créer par ailleurs d'affaissement trop prononcé ou d'écrasement de la paroi ventrale contre la paroi dorsale.

Par ailleurs, selon une caractéristique importante de l'invention, les moyens de renfort 20 peuvent s'étendre, de préférence exclusivement, à l'extérieur de la chambre 10, tel que cela est illustré sur les figures 8 et 9.

Avantageusement, une telle solution permet notamment de minimiser, voire de supprimer, le rétrécissement de la section du passage 30 lié à un empiètement desdits moyens de renfort 20 sur le volume utile de la chambre 10.

Plus particulièrement, tel que cela est représenté sur les figures 8 et 9, les moyens de renfort 20 seront formés dans le cadre de l'invention par des languettes 45 venues de matière avec ladite ceinture dorsale 16 et repliées sur les parois latérales de ladite chambre.

Bien entendu, la rigidité de la ceinture dorsale 16 sera à cet effet choisie supérieure à celle de la membrane 31. En particulier, on pourra réaliser la membrane 31 dans un élastomère biocompatible, du genre silicone, d'une dureté Shore comprise entre 14 et 35, et la ceinture dorsale 16 dans un élastomère biocompatible, du genre silicone, d'une dureté Shore comprise entre 50 et 80.

La forme de telles languettes 45 ne sera pas particulièrement limitée. Toutefois, elles se présenteront de préférence sous la forme de triangles ou de trapèzes reliés à la ceinture dorsale par leur base conférant ainsi à ladite dorsale une allure d'araignée.

En outre, la pointe desdites languettes pourra être pourvue d'un système d'encliquetage ou de blocage radial destiné à coopérer avec la paroi latérale 14, 15, dans la portion ventrale de la poche 10V, pour améliorer la « prise en tenaille» de cette dernière au droit des arêtes.

Plus particulièrement, on pourra prévoir un ergot en saillie sur la face interne cachée de la languette et destiné à se ficher dans un logement creusé dans la membrane 31.

La fixation des languettes aux parois latérales pourra se faire par collage, et éventuellement être renforcée en prévoyant dans la membrane 31 des saignées (non représentées) de forme conjuguée auxdites languettes, afin que ces dernières soient encastrées de manière affleurante ou légèrement saillante dans lesdites parois latérales 14, 15.

Un procédé de fabrication d'un anneau chirurgical 1 présentant l'une ou l'autre des caractéristiques décrites ci-dessus comportera avantageusement une étape (a) de moulage au cours de laquelle on réalisera d'un seul tenant la chambre 10 et les moyens de renfort 20, et en particulier les nervures 42 latérales appariées.

De préférence, ladite étape sera réalisée par injection en utilisant un outillage adapté et un noyau unique dont le gabarit correspond au contour intérieur de la chambre 10 et qui présente en creux dans sa surface une pluralité d'empreintes correspondant auxdites nervures 42.

Il est remarquable que l'anneau 1 conforme à l'invention peut être réalisé au moyen d'un outillage particulièrement simple à usiner, donc peu coûteux, et ne pose quasiment aucun problème de démoulage, ce qui simplifie sa fabrication et limite le taux de rebut.

Le fonctionnement d'un anneau chirurgical 1 conforme à l'invention va maintenant être décrit plus en détails.

Le praticien introduit tout d'abord un anneau chirurgical sous la peau après avoir pratiqué une incision.

Pour ce faire, il peut procéder par coelioscopie et introduire l'anneau 1 à travers une canule.

A cet effet, le praticien peut avantageusement redresser à force la bande souple 2, en utilisant son élasticité intrinsèque, pour lui conférer temporairement une forme sensiblement rectiligne.

Lorsque l'anneau 1 se trouve dans l'organisme, il tend à retrouver spontanément, par mémoire de forme, sa forme incurvée.

Le praticien peut alors l'engager autour de l'organe, puis rapprocher l'une de l'autre les extrémités 3, 4 jusqu'à les faire se toucher, de sorte à former une boucle close autour dudit organe. L'engagement des moyens de verrouillage 5, 6 maintien l'anneau en configuration fermée.

Lorsqu'il rapproche l'une de l'autre les extrémités 3, 4 de la bande souple 2, le praticien force la flexion de ladite bande souple 2, ce qui a pour effet de marquer davantage les plis formés par les arêtes, et par conséquent de refermer progressivement l'angle formé entre les facettes consécutives.

Avantageusement, les plis ainsi formés sont spontanément localisés, selon une distribution angulaire sensiblement uniforme, au niveau des zones de pliage qui forment des amorces de déformation en flexion.

Une fois l'anneau en place, les parois ventrales 11 des tronçons 22 viennent au contact de la paroi de l'organe biologique.

Le praticien peut alors introduire un fluide sous pression dans la chambre gonflable 10, et ainsi provoquer la déformation des facettes.

Plus particulièrement, il bombe lesdites facettes, c'est-à-dire les zones intermédiaires libres qui sont comprises entre les arêtes, de telle sorte que lesdites facettes forment des dômes qui enflent selon une direction radiale centripète en resserrant progressivement l'organe biologique.

Avantageusement, durant cette opération de gonflage, les nervures 42 maintiennent les arêtes en retrait des facettes, en accentuant ainsi les plis formés entre deux facettes voisines, en limitant la déformation élastique centripète sensiblement aux seules facettes, et plus particulièrement à la zone centrale de chaque facette située à équidistance des deux arêtes qui délimitent cette dernière.

Ainsi, on contrôle précisément les zones de déformation de l'anneau 1, de même que le diamètre global de la lumière dudit anneau à travers laquelle passe l'organe biologique.

Si le praticien souhaite relâcher l'étreinte exercée par l'anneau sur l'organe biologique voire extraire l'anneau, il peut procéder à une ponction de fluide dans la chambre 10 de sorte à dégonfler celle-ci.

Les dômes formés au niveau des facettes tendent alors à s'affaisser par retour élastique, de telle sorte que la paroi ventrale 11 retrouve peu à peu sa structure au repos, avec des facettes sensiblement planes.

Au besoin, le praticien peut déverrouiller l'anneau afin d'ouvrir la boucle, par exemple en s'aidant des languettes de préhension 7, 8.

Ainsi, l'anneau 1 conforme à l'invention permet d'obtenir un serrage adaptable, atraumatique, et respectueux de l'intégrité physique de l'anneau, puisqu'il limite le travail en fatigue de la membrane 31, en répartissant de manière équilibrée, prévisible et contrôlée les plis le long de la paroi ventrale 11. De surcroît, la présence de moyens de renfort contribue à conférer à la chambre 10 un volume non nul au repos, et ainsi améliore l'effet de suspension, et notamment la tenue à l'écrasement radial centrifuge de l'anneau.

En outre, un tel anneau présente une structure particulièrement simple et légère, dont le coût de fabrication est relativement modeste.

Enfin, il est remarquable que, quelque soit le nombre de cycles d'ouverture ou de fermeture dudit anneau 1, ou de gonflage/dégonflage de celui-ci, son comportement est contrôlé et reproductible, tant au niveau de la position, de l'orientation, ou de l'intensité des plis formés, que de la dilatation ou de la contraction des facettes.

## Revendications

1. Anneau chirurgical implantable (1) comportant une bande souple (2) apte à fléchir sur elle même pour former une boucle autour d'un organe biologique constituant une poche ou un conduit afin de modifier la section de passage dudit organe biologique, ladite bande souple comportant une chambre gonflable (10) délimitée par une paroi ventrale (11) destinée à venir au contact de l'organe et une paroi dorsale (12) opposée orientée vers l'extérieur de l'anneau, de telle sorte que ladite chambre (10) se répartit en une portion ventrale (10V) et une portion dorsale (10D) situées respectivement de part et d'autre d'une surface médiane fictive (M) sensiblement équidistante de la paroi ventrale (11) et de la paroi dorsale (12), ledit anneau comportant une pluralité de moyens de renfort (20) reliant chacun la portion ventrale (10V) de la chambre à la portion dorsale (10D) de celle-ci afin de limiter le débattement de la paroi ventrale (11) par rapport à la paroi dorsale (12) lors du gonflage de la chambre et ainsi créer au niveau de la paroi ventrale des zones de pliage (21) prédéfinies qui subdivisent ladite chambre en une pluralité de tronçons (22) contigus, lesdits moyens de renfort (20) s'étendant à l'extérieur de la chambre (10), lesdits moyens de renfort (20) étant agencés pour conserver entre les tronçons contigus des orifices de passage (30) permettant la libre circulation de fluide de gonflage et dont la section (SP) est supérieure ou égale à 10 %, de préférence supérieure ou égale à 30%, et de façon particulièrement préférentielle supérieure ou égale à 50%, de la section (S0) desdits tronçons (22),
**Caractérisé en ce que** la paroi ventrale (11) est formée par une membrane en élastomère (31) qui est fixée à une ceinture dorsale (16) de rigidité supérieure, les moyens de renfort (20) étant formés par des languettes (45) venues de matière avec ladite ceinture dorsale (16) et repliées sur les parois latérales de ladite chambre.

2. Anneau selon la revendication 1 **caractérisé en ce que** les moyens de renfort (20) sont formés par des tirants qui joignent la paroi ventrale (11 ) à la paroi dorsale (12).

3. Anneau selon la revendication 1 ou 2 **caractérisé en ce que** les moyens de renfort (20) sont disposés par paire de part et d'autre de la largeur de la chambre (10).

4. Anneau selon la revendication 3 **caractérisé en ce que** les moyens de renfort (20) de chaque paire sont disposés de manière sensiblement symétrique par rapport au plan sagittal (P) de la chambre (10).

5. Anneau selon une des revendications précédentes **caractérisé en ce que** les moyens de renfort (20) comportent des nervures (42) formant des surépaisseurs venues de matière avec les parois latérales (14, 15) de la chambre (10).

6. Anneau selon l'une des revendications précédentes **caractérisé en ce que** les moyens de renfort (20) s'étendent sensiblement perpendiculairement à la face ventrale (11) et à la face dorsale (12).

7. Anneau selon l'une des revendications 1 à 6 **caractérisé en ce que** les moyens de renfort (20) sont disposés en oblique de sorte à former des structures triangulaires dont les sommets coïncident avec les zones de pliage (21).

8. Anneau selon l'une des revendications précédentes **caractérisé en ce que** les moyens de renfort (20) s'étendent en saillie à l'intérieur de la chambre (10).

9. Anneau selon l'une des revendications précédentes **caractérisé en ce qu'**il constitue un anneau de by-pass gastrique.

## Claims

1. Implantable surgical ring (1) comprising a flexible band (2) able to bend back on itself in order to form a loop around a biological organ constituting a pouch or a conduit, so as to modify the cross section of flow through said biological organ, said flexible band comprising an inflatable chamber (10) delimited by a ventral wall (11), which is intended to come into contact with the organ, and an opposite dorsal wall (12), which is oriented to the outside of the ring, such that said chamber (10) divides into a ventral portion (10V) and a dorsal portion (10D), which are respectively situated on each side of an imaginary median surface (M) substantially equidistant from the ventral wall (11) and the dorsal wall (12), said ring comprising a plurality of reinforcement means (20), which each connect the ventral portion (10V) of the chamber to the dorsal portion (10D) of the latter, so as to limit the clearance of the ventral wall (11) with respect to the dorsal wall (12) during the inflation of the chamber and thereby create, in the area of the ventral wall, predefined folding zones (21) that subdivide said chamber into a plurality of contiguous segments (22), said reinforcement means (20) extending outside the chamber (10), said reinforcement means (20) being designed to maintain, between the contiguous segments, through-orifices (30) which permit the free circulation of inflation fluid and of which the cross section (SP) is greater than or equal to 10%, preferably greater than or equal to 30%, and more particularly preferably greater than or equal to 50%, of the cross section (SO) of said segments (22), **characterized in that** the ventral wall (11) is formed by an elastomeric membrane (31), which is fixed to a dorsal belt (16) of greater rigidity, the reinforcement means (20) being formed by tabs (45), which are produced integrally with said dorsal belt (16) and are folded back on the lateral walls of said chamber.

2. Ring according to Claim 1, **characterized in that** the reinforcement means (20) are formed by struts which join the ventral wall (11) to the dorsal wall (12).

3. Ring according to Claim 1 or 2, **characterized in that** the reinforcement means (20) are arranged in pairs on each side of the width of the chamber (10).

4. Ring according to Claim 3, **characterized in that** the reinforcement means (20) of each pair are arranged substantially symmetrically with respect to the sagittal plane (P) of the chamber (10).

5. Ring according to one of the preceding claims, **characterized in that** the reinforcement means (20) comprise ribs (42) forming areas of increased thickness which are produced integrally with the lateral walls (14, 15) of the chamber (10).

6. Ring according to one of the preceding claims, **characterized in that** the reinforcement means (20) extend substantially perpendicularly with respect to the ventral face (11) and the dorsal face (12).

7. Ring according to one of Claims 1 to 6, **characterized in that** the reinforcement means (20) are arranged obliquely, so as to form triangular structures whose vertices coincide with the folding zones (21).

8. Ring according to one of the preceding claims, **characterized in that** the reinforcement means (20) extend protruding inside the chamber (10).

9. Ring according to one of the preceding claims, **characterized in that** it constitutes a gastric bypass ring.

## Patentansprüche

1. Implantierbarer chirurgischer Ring (1), der ein elastisches Band (2) aufweist, das sich auf sich selbst umbiegen kann, um eine Schlaufe um ein biologisches Organ zu formen, die eine Tasche oder einen Kanal formt, um den Durchgangsquerschnitt des biologischen Organs zu verändern, wobei das elastische Band eine aufpumpbare Kammer (10) aufweist, die durch eine Bauchwand (11), die dazu bestimmt ist, mit dem Organ in Kontakt zu kommen, und durch eine gegenüberliegende Rückenwand (12) definiert wird, die zur Außenseite des Rings gerichtet ist, so dass die Kammer (10) sich in einen Bauchteil (10V) und einen Rückenteil (10D) aufteilt, die je zu beiden Seiten einer fiktiven Mittelfläche (M) angeordnet sind, die im Wesentlichen den gleichen Abstand zur Bauchwand (11) und zur Rückenwand (12) hat, wobei der Ring eine Vielzahl von Verstärkungseinrichtungen (20) aufweist, die je den Bauchteil (10V) der Kammer mit deren Rückenteil (10D) verbinden, um den Bewegungsraum der Bauchwand (11) bezüglich der Rückenwand (12) beim Aufpumpen der Kammer zu begrenzen und so im Bereich der Bauchwand vordefinierte Faltzonen (21) zu bilden, die die Kammer in eine Vielzahl von aneinandergrenzenden Abschnitten (22) unterteilen, wobei die Verstärkungseinrichtungen (20) sich außerhalb der Kammer (10) erstrecken, wobei die Verstärkungseinrichtungen (20) eingerichtet sind, um zwischen den aneinandergrenzenden Abschnitten Durchgangsöffnungen (30) beizubehalten, die den freien Kreislauf des Aufpumpfluids erlauben und deren Querschnitt (SP) größer als oder gleich 10%, vorzugsweise größer als oder gleich 30%, und besonders bevorzugt größer als oder gleich 50% des Querschnitts (S0) der Abschnitte (22) ist,
**dadurch gekennzeichnet, dass** die Bauchwand (11) von einer Elastomermembran (31) geformt wird, die an einem Rückengurt (16) höherer Steifheit befestigt ist, wobei die Verstärkungseinrichtungen (20) von Zungen (45) geformt werden, die aus dem gleichen Material wie der Rückengurt (16) bestehen und auf die Seitenwände der Kammer umgebogen sind.

2. Ring nach Anspruch 1, **dadurch gekennzeichnet, dass** die Verstärkungseinrichtungen (20) von Zugstange geformt werden, die die Bauchwand (11) mit der Rückenwand (12) verbinden.

3. Ring nach Anspruch 1 oder 2, **dadurch gekennzeichnet, dass** die Verstärkungseinrichtungen (20) paarweise zu beiden Seiten der Breite der Kammer (10) angeordnet sind.

4. Ring nach Anspruch 3, **dadurch gekennzeichnet, dass** die Verstärkungseinrichtungen (20) jedes Paars im Wesentlichen symmetrisch bezüglich der Sagittalebene (P) der Kammer (10) angeordnet sind.

5. Ring nach einem der vorhergehenden Ansprüche, **dadurch gekennzeichnet, dass** die Verstärkungseinrichtungen (20) Rippen (42) aufweisen, die Überdicken bilden, die aus einem Material mit den Seitenwänden (14, 15) der Kammer (10) bestehen.

6. Ring nach einem der vorhergehenden Ansprüche, **dadurch gekennzeichnet, dass** die Verstärkungseinrichtungen (20) sich im Wesentlichen lotrecht zur Bauchseite (11) und zur Rückenseite (12) erstrecken.

7. Ring nach einem der Ansprüche 1 bis 6, **dadurch gekennzeichnet, dass** die Verstärkungseinrichtungen (20) schräg angeordnet sind, um dreieckige Strukturen zu formen, deren Spitzen mit den Faltzonen (21) zusammenfallen.

8. Ring nach einem der vorhergehenden Ansprüche, **dadurch gekennzeichnet, dass** die Verstärkungseinrichtungen (20) sich ins Innere der Kammer (10) vorstehend erstrecken.

9. Ring nach einem der vorhergehenden Ansprüche, **dadurch gekennzeichnet, dass** er einen Ring eines Magenbypasses bildet.
